Europäisches Patentamt

(19) European Patent Office

Office européen des brevets

(11) Publication number: **0 085 658**
A2

## (12) EUROPEAN PATENT APPLICATION

(21) Application number: 83830064.8

(51) Int. Cl.³: **A 61 B 17/38**

(22) Date of filing: 23.03.83

(30) Priority: 05.04.82 US 365232

(43) Date of publication of application:
10.08.83 Bulletin 83/32

(84) Designated Contracting States:
AT BE CH GB LI LU NL SE

(71) Applicant: Jacobsen, Stephen C.
274 South, 1200 East
Salt Lake City Utah 84102(US)

(71) Applicant: Hanover, Barry K.
558 South, 1000 East
Salt Lake City Utah 84102(US)

(71) Applicant: Tanner, Howard M.
1725 South, 800 East
Salt Lake City Utah 84105(US)

(72) Inventor: Jacobsen, Stephen C.
274 South, 1200 East
Salt Lake City Utah 84102(US)

(72) Inventor: Hanover, Barry K.
558 South, 1000 East
Salt Lake City Utah 84102(US)

(72) Inventor: Tanner, Howard M.
1725 South, 800 East
Salt Lake City Utah 84105(US)

(74) Representative: Tonon, Gilberto et al,
c/o Società Italiana Brevetti Piazza Poli 42
I-00187 Roma(IT)

(54) Electro-surgical cutting and cauterizing tool.

(57) A disposable electro-surgical cutting and cauterizing tool is provided as a hand held tool for regulating the applications of electric power impulses or signals to an electro-surgical electrode. Cutting and coagulation signals can be selectively applied by the actuation of switches in a handle supporting the electrode. The handle of the tool is shaped to comfortably conform to the human hand and contains an index finger operated tactile switch for actuating the tool in either a cutting or coagulation mode of operation. The tool is made simple and inexpensive and is sterilized during packaging. It is disposable so that re-sterilization expenses are avoided. A reversible tip is provided to permit the user to select the best operating tool.

FIG.2

Jacobsen, Stephen C.

Hanover, Barry K.

Tanner, Howard M.

## ELECTRO-SURGICAL CUTTING AND CAUTERIZING TOOL

### Background of the invention

The present invention relates to electro-surgical tools that are used for cutting and cauterizing or coagulation procedures.

It is well known in the prior art to use various types of high frequency current in electro-surgical tools to perform medical procedures such as cauterization of blood vessels and cutting of tissue.

When an electro-surgical tools is used, the actual cutting of tissue is accomplished by the concentrated application of high frequency electrical energy which destroys the tissue cells directly beneath the electro-surgical electrode of the tool. Coagulation and cauterization is accomplished by the shrinking of blood vessels and by effecting the coagulation of blood fluid through heating the blood in the immediate proximity of the electrode.

It is common for a doctor to accomplish both cutting and coagulation/cauterization procedures alternatively during a single medical operation. Accordingly, it is desirable to have a switching mechanism to change operation from a cutting signal to a coagulation signal and vice versa. Preferably, the switching should occur in a manner that requires minimum diversion of attention by the physician and that provides for positive knowledge on the part of the physician that switching has occurred. The tool should positively prevent the presence of a signal for cutting at the electrode when the switch means is positioned for cauterization, and vice versa.

In addition to accomplishing the selection of mode of operation of the electro-surgical tool with a minimum of diversion, it is desirable for the user to positively know when the device has power to the electrode and to be able to not only select a mode of operation, but also, to have the power to the electrode automatically turned off when an operating mode has not been selected.

The switching means employed in previously known electro-surgical tools are often awkward to use and may prevent the obtaining of the high degree of precision needed in many medical procedures. It has been determined that an electro-surgical knife should have the switching control on the knife handle itself and should have switching means that can be operated quickly and with minimum effort to give a positive tactile switching.

Some electro-surgical tools of the prior art have been made with some disposable and replaceable components. U.S. patent 2310844 discloses an electro-surgical tool that utilizes a replaceable electrode and U.S. patent 4034761 discloses such a tool having a replaceable handle. While some advantages may be realized as a result of using replaceable components, it still becomes necessary to sterilize all elements that are to be repeatedly used. Consequently, it is desirable to have a unit that is totally disposable, even including the power cord. Total disposability is only practical if the entire device can be made inexpensively. Electrodes of different styles can be used in those known tools that provide for electrode replacement.

Many devices disclosed in the prior art are bulky and heavy. These are not entirely satisfactory since for precise medical procedures the electro-surgical tool should fit the human hand comfortably and should be very light to avoid tiring the user even during long medical procedures.

Electro-surgical tools of various types are disclosed in U.S. patents 3974833 (Durden; 4170 234 (Graham); 4202337 (Hren, et al); 4034761 (Prater); 4112950 (Pike); 3648001 (Anderson, et al); 2310844 (Draeger).

It is a principal object of the present invention to provide an electro-surgical tool that is sufficiently inexpensive to build, that can be economically used once, and then be totally discarded.

Another object of the present invention is to provide an electro-surgical tool that fits the human hand securely and comfortably.

Still another object of the present invention is to provide an electro-surgical tool that provides a positive tactile indication to a user when it is in an operating mode and that indicates the mode currently in use.

Yet another object of the present invention is to provide an electro-surgical tool wherein the power to the device is conveniently controlled by the same hand that is used to manipulate the device.

Another object of the invention is to provide an electro-surgical tool that is light in weight.

Another object is to provide a surgical tool including an electrode that is reversible to give different electrode styles as desired by the user thereof.

Principal features of the invention include an electrode holder forming a handle and made of a rigid plastic material. Axial openings are provided at each end of the handle. Electrical conductors enter through the rear axial opening and extend substantially the length of the inside of the holder. The electrical conductors are connected to two switches, one of which controls current providing a cutting signal and the other of which controls current providing a coagulation/cauterization signal to an electrode. Membrane switches are used to achieve light weight, low cost, and a positive tactile contact by the user. The switches function as spring return to normal devices. The switch buttons are preferably different colors to assist a user in differentiation. The electrode is made with a flat section and a round section, the sections being angled with respect to one another and with each section being adapted to be inserted into a socket electrically connected to the switches.

Other objects and features of the invention will become apparent from the following detailed description and drawing disclosing what is presently contemplated as being the best mode of the invention:

In the drawing:

fig. 1 is a side view of the electro-surgical tool of the invention;

fig. 2 is a longitudinal section view of the tool;

fig. 3 an end front view of the tool; and

fig. 4 a wiring diagram of the tool.

## Detailed description of the invention

Referring now to the drawing:

In the illustrated preferred embodiment, the electro-surgical cutting and cauterizing tool of the invention is shown generally at 10.

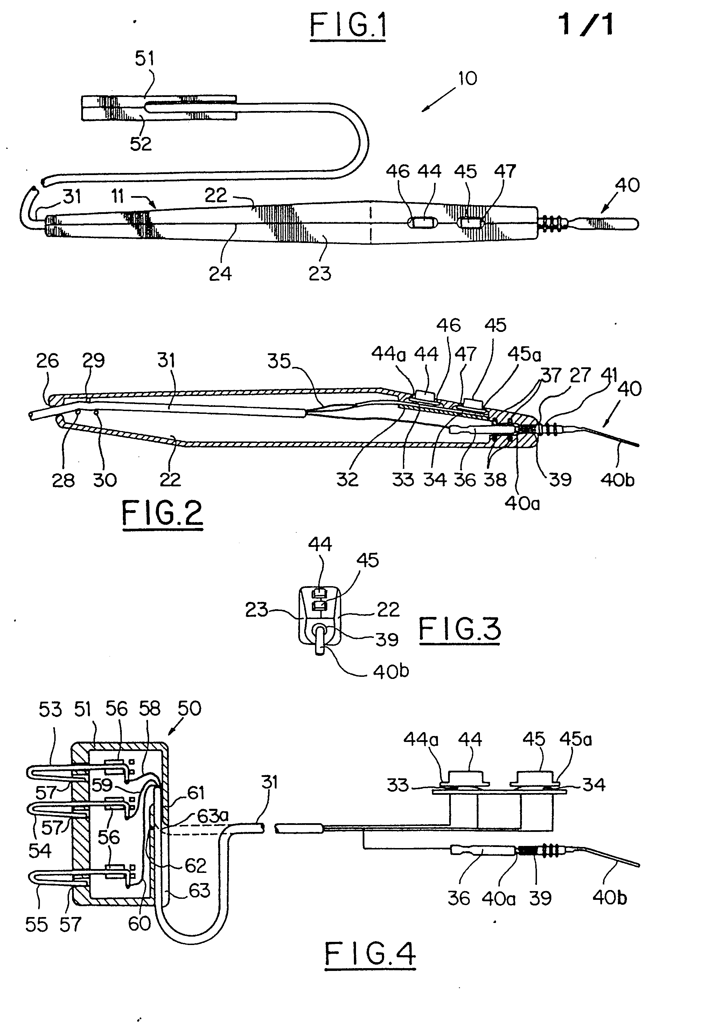

As shown best in figures 1 and 2, the tool includes an electrode holder 11 that also serves as a handle for the tool.

The electrode holder 11 is made from a pair of elongate sections 22 and 23 that are bonded together at 24 to have a body cavity formed between them and with rear and front openings 26 and 27 at opposite ends thereof. The section 22 has three pins 28, 29 and 30 projecting inwardly near the rear opening 26 to engage the section 23 and to provide means by which an electrical cord 31 passed through the opening 26 is wedged against being pulled from the holder.

A platform 32 also projects inwardly from the section 22 to engage the section 23 and to provide a rest for a pair of membrane switches 33 and 34. The membrane switches are electrically connected by small staples 35 in conventional fashion, to the electrical cord 31 and to a metal socket 36. The metal socket is wedged between upper and lower posts 37 and 38, respectively, to be securely held in the holder 11.

A splined barrel 39 is fixed in the opening 27 at the front of holder 11 and the electrode 40, which serves as the cutter and cauterizer of the tool, has a similarly splined insulating collar 41 thereon, adapted to be tightly fitted into the splined barrel 39 when the electrode is inserted into socket 36. The spline connection between barrel 39 and collar 41 then prevents rotation of the electrode 40 in multiple positions with respect to the holder 11.

Electrode 40 includes a shaft 40a of circular cross-sectional configuration and a flat portion 40b angled with respect to shaft 40a. Either shaft 40a or flat portion 40b may be inserted into socket 36 to make a good electrical connection through the socket with the switches. The shaft or flat portion not inserted in the socket will then project from the tool to be used in surgical procedures. The electrode is thus reversible and the user may select the electrode style preferred for the surgical procedure to be undertaken.

Push buttons 44 and 45 are respectively positioned on top of the membrane switches 33 and 34 and extend through openings 46 and 47 in the top of the holder. Bases 44a and 45a on the buttons are too large to pass through the openings 46 and 47 and provide surfaces against which the membrane switches act to raise the push buttons to normally "off" positions. The push buttons must be forcefully pushed down against the bias of the membrane switches to operate the switches to activate circuits to the electrode.

A low cost electrical connector 50 is attached to the end of cord 31 opposite the holder 11. The connector 50 is made of two flat housing sections 51 and 52 that are snapped together with connector pins 53, 54 and 55 (fig. 4) projecting therefrom to be connected into an outlet of a high frequency electrical generator, not shown. The connector pins 53, 54 and 55 are bent hairpin-type pins, each having one end fixed in a holder 56 formed in section 51 and the other end constrained for movement in an opening 57. Lead wires 58, 59 and 60 from cord 31 are respectively connected to the fixed ends of the connector pins 53, 54 and 55 and the cord 31 is positioned in a groove 61 extending to the top center of the connector and along a ridge 62 so that the cord will normally hang down from the connector. If pulled outwardly however, the cord will extend from a terminal end 63a of slot 63 to provide even pulling of the connector 50 from the generator.

As illustrated best in fig. 4, power circuits are established to the electrode 40 from connector pin 54 through either switch 33 and connector pin 53 or switch 34 and connector pin 55.

The holder 11 provides a pencil-like configuration adapted to comfortably rest between the thumb and the forefinger of the user's hand and such that the tip of the forefinger will easily reach either of the buttons 44 or 45.

Because the holder 11 and connector 50 are both easily and inexpensively formed and since all components are inexpensive to obtain and/or produce and since the assembly of the components is easily and quickly performed, the overall cost of the surgical tool of the invention is such that it can be economically used once and then the entire unit including the electrode 40, holder 11, connector 50 and cord 31 can be discarded.

Although a preferred embodiment of our invention is herein described, it is to be understood that the present disclosure is by way of example and that variations are possible without departing from the subject matter coming within the scope of the following claims, which subject matter we regard as our invention.

Claims

1.    A disposable electro-surgical cutting and cauterizing tool comprising an electrode holder of elongate
configuration, having openings at opposite ends thereof;
an electrical cord having one end extending through one
end of the electrode holder and into the holder; a pair
of spaced apart normally biased off electrical switch
means projecting through a top surface of the electrode
holder adjacent to other end of the holder; an electrode
electrically connected to the electrical cord and to
the switch means and projecting from the other end of
the holder; and a flat connector at the other end of the
cord, said connector having connector pins projecting
from one edge of the connector and means connecting the
other end of the cord into the connector at an opposite
edge thereof.

2.    A disposable electro-surgical tool as in claim 1,
wherein the switch means each include a membrane switch
and a push button resting on the membrane switch and the
electrode holder has a platform thereon to position the
membrane switches beneath the push buttons.

3.    A disposable electro-surgical tool as in claim 1 or
2,further including an electrically conductive socket in
the holder, said socket being connected to the electrical
cord and receiving one end of the electrode, and means in
the holder for preventing rotation of the electrode.

4.    A disposable electro-surgical tool as in any of the
preceding claims, wherein the end of the electrode remote from the holder is angled with respect to the longitudinal axis of the holder.

5.    A disposable electro-surgical tool as in any of
the preceding claims, wherein the electrode holder
has a pencil-like configuration and is tapered from
the top surface to a bottom surface.

6.    A disposable electro-surgical tool as in any of
the preceding claims, wherein the end of the cord
extends into the connector parallel to a slot
extending to near the mid-point of the connector along
the opposite edge thereof.

7.    A disposable electro-surgical tool as in any of
the preceding claims, wherein the electrode is
reversible in the holder to present for use, a pair
of differently shaped surgical tools.

8.    A disposable electro-surgical tool, substantially
as previously described with reference to the figures
of enclosed drawing.

FIG.1

FIG.2

FIG.3

FIG.4